(19) **Europäisches Patentamt**
**European Patent Office**
**Office européen des brevets**

(11) **EP 2 878 263 A1**

(12) **EUROPEAN PATENT APPLICATION**
published in accordance with Art. 153(4) EPC

(43) Date of publication:
**03.06.2015 Bulletin 2015/23**

(51) Int Cl.:
*A61B 5/18* (2006.01)   *A61B 5/0245* (2006.01)
*B60R 16/02* (2006.01)

(21) Application number: **13822977.8**

(22) Date of filing: **14.06.2013**

(86) International application number:
**PCT/JP2013/003747**

(87) International publication number:
**WO 2014/017009 (30.01.2014 Gazette 2014/05)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **26.07.2012 JP 2012166225**

(71) Applicant: **Nissan Motor Co., Ltd.**
**Yokohama-shi**
**Kanagawa 221-0023 (JP)**

(72) Inventors:
• **MORI, Souichirou**
**Atsugi-shi**
**Kanagawa 243-0123 (JP)**

• **SHIMIZU, Youji**
**Atsugi-shi**
**Kanagawa 243-0123 (JP)**
• **MIURA, Hiroaki**
**Atsugi-shi**
**Kanagawa 243-0123 (JP)**
• **FUKUYAMA, Yasuhiro**
**Atsugi-shi**
**Kanagawa 243-0123 (JP)**
• **SUNDA, Takashi**
**Atsugi-shi**
**Kanagawa 243-0123 (JP)**

(74) Representative: **Osha Liang**
**2, rue de la Paix**
**75002 Paris (FR)**

(54) **DRIVER STATE ESTIMATION DEVICE AND DRIVER STATE ESTIMATION METHOD**

(57) To improve an estimate accuracy in estimating a steady state of a driver. A heart rates R of the driver are recorded and the deceleration start time tg when a vehicle in a travelling state starts to decelerate and stops. The heart rates R within a predetermined period recorded before the deceleration start time tg by the biological information record unit (21) are referred to and a pre-deceleration heart rate Rb which is a representative value of the referred heart rates R is extracted. Furthermore, the heart rates R within a predetermined period recorded after the deceleration start time tg by the biological information record unit (21) are referred to and a post-deceleration heart rate Ra which is a representative value of the referred heart rates R is extracted. Then, the pre-deceleration biological information and the post-deceleration biological information extracted by the biological information extraction unit are compared with each other to estimate the steady state of the driver after deceleration. That is, when a difference ΔR between the pre-deceleration heart rate Rb and the post-deceleration heart rate Ra is equal to or larger than a predetermined threshold value Rt, it is estimated that the driver after the deceleration in in the steady state.

FIG. 4A
FIG. 4B
FIG. 4C
FIG. 4D

**Description**

Technical Field

This disclosure relates to a driver's state estimation apparatus and a driver's state estimation method.

Background Art

PTL 1 discloses a navigation apparatus which searches a facility around the current location and provides information thereof. The navigation apparatus measures biological information of a passenger, such as a blood-pressure, a pulse rate, a body temperature, or the like, and provides facility information depending on a health condition of the passenger.

Citation List

Patent Literature

PTL1: JP 2007-47196

Summary of Invention

Technical Problem

With regard to the blood-pressure as one example of the biological information, a normal value can be obtained only when a measuring object person is in a steady state. For example, the blood-pressure generally becomes higher after exercise, after meals, on smoking, at a cold time, or the like. The blood-pressure also generally becomes higher when the person is in an excited state due to emotional ups and downs, or in a nervous state. Therefore, in order to obtain the normal value of the blood-pressure, it is firstly necessary to know whether or not the measuring object person is in the physically and mentally steady state. However, it is difficult to estimate that the measuring object person is in the steady state. Especially, the blood-pressure of the driver fluctuates largely during travelling, and the driver is not always in the steady state even when a vehicle is stopped.

One object of an embodiment of the present invention is to improve an estimate accuracy in estimating the driver's steady state.

According to one embodiment of the present invention, there is provided a driver's state estimation apparatus in which biological information of a driver is recorded and a deceleration start time is detected when a vehicle in travelling state starts to decelerate and stops. Then, the biological information within a predetermined period recorded before the deceleration start time is referred to and pre-deceleration biological information which is a representative value of the referred biological information is extracted, and the biological information within a predetermined period recorded after the deceleration start time is referred to and post-deceleration biological information which is a representative value of the referred biological information is extracted. Then, the extracted pre-deceleration biological information and the extracted post-deceleration biological information are compared with each other to estimate whether or not the driver after deceleration is in a steady state.

Advantageous Effects of Invention

According to one embodiment of the present invention, the pre-deceleration biological information before the deceleration start time and the post-deceleration biological information after the deceleration start time are compared with each other to estimate whether or not the driver after the deceleration is in the steady state. Therefore, it is possible to improve an estimate accuracy. That is, focusing on a tendency of the biological information of the driver to be stabilized uniformity after the change of the biological information of the driver due to the deceleration of the vehicle, it is possible to estimate whether or not the driver after the deceleration is in the steady state by comparing the pre-deceleration biological information and the post-deceleration biological information.

Brief Description of Drawings

FIG. 1 is a schematic configuration of a driver's state estimation apparatus;

FIG. 2 is a block diagram illustrating a driver's state estimation processing;

FIG. 3 is a time chart illustrating a pre-deceleration comparison period Tb and a post-deceleration comparison period Ta;

FIG. 4A to FIG. 4D are views illustrating an estimation of a steady state;

FIG. 5 is a flowchart illustrating a driver's state estimation processing;

FIG. 6 is a time chart illustrating a reaction time Tx and a deceleration start time tg;

FIG. 7 is a block diagram illustrating a driver's state estimation processing of a second embodiment;

FIG. 8A and FIG. 8B are views illustrating a calculation method of a blood-pressure value P;

FIG. 9 is a time chart illustrating a post-deceleration stability period Ts;

FIG. 10 is a flowchart illustrating a driver's state estimation processing of the second embodiment;

FIG. 11 is a block diagram illustrating a driver's state estimation processing of a third embodiment; and

FIG. 12 is a flowchart illustrating a driver's state estimation processing of the third embodiment.

Description of Embodiments

Hereinafter, embodiments of the present invention will be described with reference to the drawings.

(First Embodiment)

(Configuration)

The present embodiment estimates a steady state of a driver.

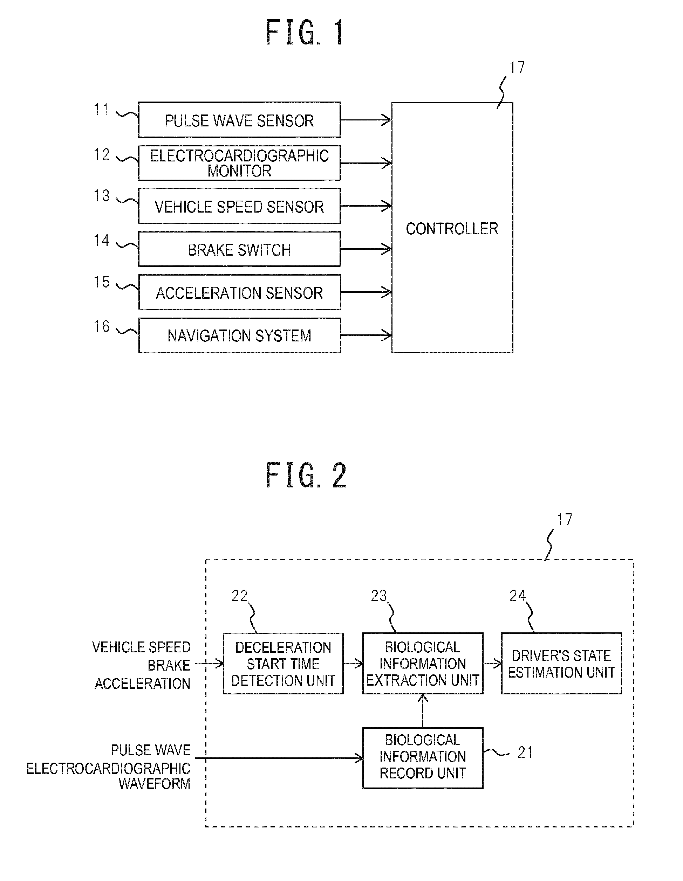

FIG. 1 is a schematic configuration of a driver's state estimation apparatus.

The driver's state estimation apparatus is installed in a vehicle and includes a pulse wave sensor 11, an electrocardiographic monitor 12, a vehicle speed sensor 13, a brake switch 14, an acceleration sensor 15, a navigation system 16, and a controller 17.

The pulse wave sensor 11 is configured to detect a pulse wave (volume pulse wave) of the driver. The pulse wave is biological information representing a change of a volume of the peripheral arterial vessel associated with the pulsation of the heart. The pulse wave sensor 11 may be formed by using a photoelectric sensor having a combination of a light emitting element and a light receiving element of the infrared light to utilize a characteristic of the near-infrared wavelength light passing through a living body and being absorbed into hemoglobin in blood. Specifically, the pulse wave sensor 11 may be provided on a grip portion of a steering wheel, for example, and configured to irradiate the infrared light to a palm or a fingertip of the driver and input a voltage signal depending on a light intensity of a scattered light reflecting thereon to the controller 17. The controller 17 configured to determine the pulse wave on the basis of the input voltage signal.

The electrocardiographic monitor 12 is configured to detect an electrocardiographic waveform of the driver. The electrocardiographic monitor 12 may include, for example, plural electrodes provided on a seating face or a surface of a backrest of a seat, or the like. The electrocardiographic monitor 12 may be configured to input to the controller 17 a potential difference signal which occurs between the respective electrodes due to a depolarization produced with a conduction of impulse which occurs when the heart muscle of the driver expands and contracts. The electrocardiographic monitor 12 is configured to determine the electrocardiographic waveform on the basis of the input potential difference. It is noted that clothes or a cloth intervenes between a skin of the driver and the electrodes, therefore, the Laplacian electrode arrangement may be employed or a capacitive measurement circuit may have a high input impedance.

The driver's state estimation apparatus of the present embodiment may be supposed to be installed in the vehicle, and the biological information may be continuously acquired without binding the driver. Therefore, the pulse wave sensor 11 and the electrocardiographic monitor 12 may be provided on the grip portion of the steering wheel or on the seating face or the surface of the backrest of the seat. The pulse wave sensor 11 and the electrocardiographic monitor 12 may be provided at a part which the driver operates within the reach of a portion of the body or a part supporting the body of the driver, as long as the biological information of the driver can be acquired.

The vehicle speed sensor 13 is configured to detect a speed of a vehicle body (hereinafter, referred to as "vehicle speed") V. The vehicle speed sensor 13 may be provided on a driven gear on an output side of a transmission, for example, and be configured to detect magnetic field lines of a sensor rotor by a detection circuit to convert the change of the magnetic field due to a rotation of the sensor rotor into a pulse signal and input it to the controller 17. The controller 17 is configured to determine the vehicle speed V on the basis of the input pulse signal.

The brake switch 14 is configured to detect ON/OFF of a brake. The brake switch 14 is configured to input a voltage signal depending on the ON/OFF of the brake to the controller 17, for example, by the use of a detection circuit including a normally-closed contact. The controller 17 is configured to determine the ON/OFF of the brake on the basis of the input voltage signal.

The acceleration sensor 15 is configured to detect an acceleration/deceleration G in a front-rear direction of the vehicle. The acceleration sensor 15 is configured to detect a displacement of a movable electrode with respect to a fixed electrode as a change of an electrostatic capacity, for example, and to convert it into a voltage signal proportional to the acceleration/deceleration and the direction to input the voltage signal to the controller 17. The controller 17 is configured to determine the acceleration/deceleration G on the basis of the input voltage signal.

The navigation system 16 is configured to recognize a current location of the vehicle and road map information at the current location. The navigation system 16 includes a GPS receiver and is configured to recognize the location (latitude, longitude, altitude) and the travelling direction of the vehicle on the basis of time differences between radio waves arriving from three or more GPS satellites. Then, the navigation system 16 is configured to refer to the road map information including road types, road shapes, widths of lanes, passing directions of vehicles, and the like, which are stored in a DVD-ROM drive or a hard disk drive, and to recognize the road map information at the current location of the vehicle to input it to the controller 17. It is noted that the Dedicated Short Range Communication (DSRC) may be used to receive various data from an infrastructure as the Driving Safety Support Systems (DSSS).

It is noted that the controller 17 is configured to directly receive the various detection signals from the sensors or the like, but is not limited thereto. The controller 17 may be connected to another control unit to receive the various data, for example, via Controller Area Network (CAN) using a multiplex communication of a CSMA/CA system.

The controller 17 may include a micro computer for example, and be configured to perform a driver's state estimation processing every predetermined period (10 msec, for example).

FIG. 2 is a block diagram illustrating the driver's state estimation processing.

The driver's state estimation processing performed by the controller 17 includes a biological information record unit 21, a deceleration start time detection unit 22, a biological information extraction unit 23, and a driver's state estimation unit 24.

The biological information record unit 21 is configured to record a vital sign of the circulatory system of the driver, which is influenced by the autonomic nerve, as the biological information. The heart rate R is the number of the pulsation of the heart within a certain period, and generally indicates the number of the pulsation per minute. Specifically, the heart rate R is calculated on the basis of the pulse wave detected by the pulse wave sensor 11 and the electrocardiographic waveform detected by the electrocardiographic monitor 12, and the heart rate R is recorded as the biological information. The biological information record unit 21 is configured to accumulate the heart rates R with time information in a nonvolatile memory and manage them.

Herein, the calculation of the heart rate R will be described.

In the present embodiment, a period of the pulsation propagating from the pumping of the heart to the periphery is measured. For example, the heart rate (sec) is calculated by dividing a unit time (60 sec, for example) by a period from a peak value to a next peak value in the pulse wave or the electrocardiographic waveform (a period of one pulsation).

What is described above is the record processing of the heart rate R by the biological information record unit 21.

The deceleration start time detection unit 22 is configured to detect a deceleration start time tg when the vehicle in a travelling state starts to decelerate and stops. Specifically, the deceleration start time detection unit 22 is configured to read the vehicle speed V detected by the vehicle speed sensor 14, the ON/OFF state and the like detected by the brake switch 14. Then, the deceleration start time detection unit 22 is configured to detect the time when the deceleration operation of the driver switches to ON as the deceleration start time tg, when the break including an engine brake switches from OFF to ON in a state where the vehicle speed V is faster than zero, and then the vehicle speed V decreases to zero. The deceleration start time tg substantially corresponds to the time when the driver recognizes that the vehicle needs to stop.

The biological information extraction unit 23 is configured to refer to the heart rates R within a predetermined pre-deceleration comparison period Tb recorded by the biological information record unit 21 before the deceleration start time tg with reference to the deceleration start time tg detected by the deceleration start time detection unit 22 to extract a pre-deceleration heart rate Rb, which is a representative value of the referred heart rates R. Furthermore, the biological information extraction unit 23 is configured to refer to the heart rates R within a predetermined post-deceleration comparison period Ta recorded by the biological information record unit 21 after the deceleration start time tg to extract a post-deceleration heart rate Ra, which is a representative value of the referred heart rates R.

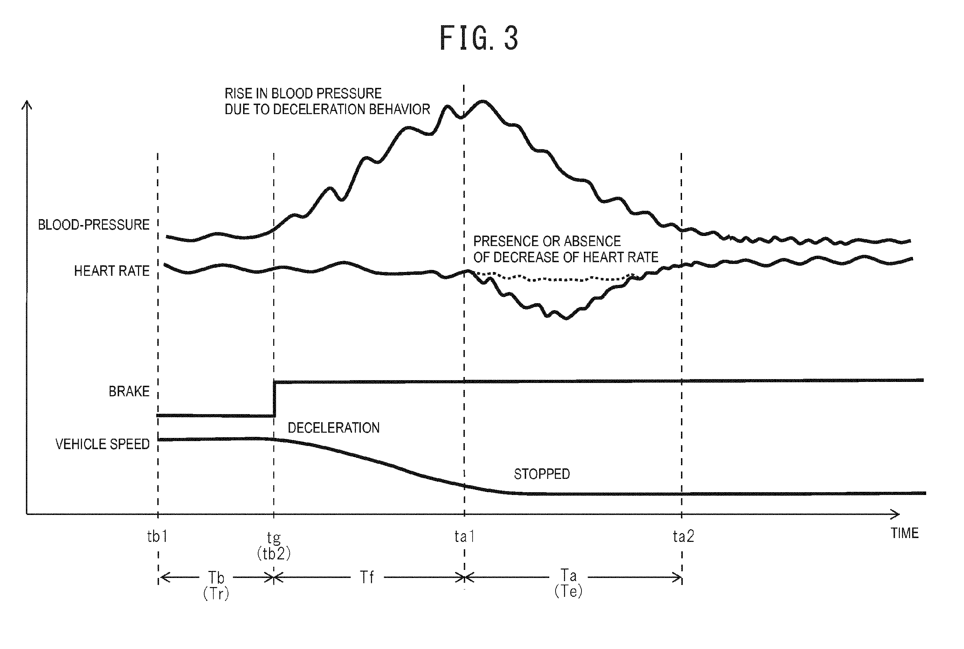

FIG. 3 is a time chart illustrating the pre-deceleration comparison period Tb and the post-deceleration comparison period Ta.

First, the time (tg-Tr=tb1) prior to the deceleration start time tg by a predetermined time Tr is set as a pre-deceleration reference start time tb1 and the deceleration start time tg is set as a pre-deceleration reference end time tb2 (tg=tb2). Then, a period from the pre-deceleration reference start time tb1 to the pre-deceleration reference end time tb2 is set as the pre-deceleration comparison period Tb (tb2-tb1=Tb=Tr).

Furthermore, the time (tg+Tf=ta1) when a predetermined time Tf elapses from the deceleration start time tg is set as a post-deceleration reference start time ta1 and the time (ta1+Te=ta2) when a predetermined time Te elapses from the post-deceleration reference start time ta1 is set as a post-deceleration reference end time ta2. Herein, Tf corresponds to a time from the start of the deceleration to the start of decrease of the heart rate R. Te corresponds to a time from the start of decrease of the heart rate R until the heart rate R returns to the same status as before the start of the deceleration. Then, the period (ta2-ta1=Ta=Te) from the post-deceleration reference start time ta1 to the post-deceleration reference end time ta2 is set as the post-deceleration comparison period Ta.

Then, a data group of the heart rates R recorded within the pre-deceleration comparison period Tb is referred to and an average value of the heart rates R is extracted as the pre-deceleration heart rate Rb, as follows.

```
Rb = Σ R (i) /N, where i=R1, R2, ..., RN
```

Furthermore, a data group of the heart rates R recorded within the post-deceleration comparison period Ta is referred to and an average value of the heart rates R is extracted as the post-deceleration heart rate Ra, as follows.

```
Ra = Σ R (i) /N, where i=R1, R2, ..., RN
```

It is noted that the data group of the heart rates R is referred to and the average value is utilized as the representative value of the referred heart rates R in the present embodiment, but the present disclosure is not limited thereto. In other embodiments, an intermediate value or a maximum value may be used as the representative value.

What is described above is the extraction processing of the pre-deceleration heart rate Rb and the post-deceleration heart rate Ra by the biological information extraction unit 23.

The driver's state estimation unit 24 is configured to compare the pre-deceleration heart rate Rb and the post-deceleration heart rate Ra extracted by the biological information extraction unit 23 to estimate the steady state of the driver after the deceleration.

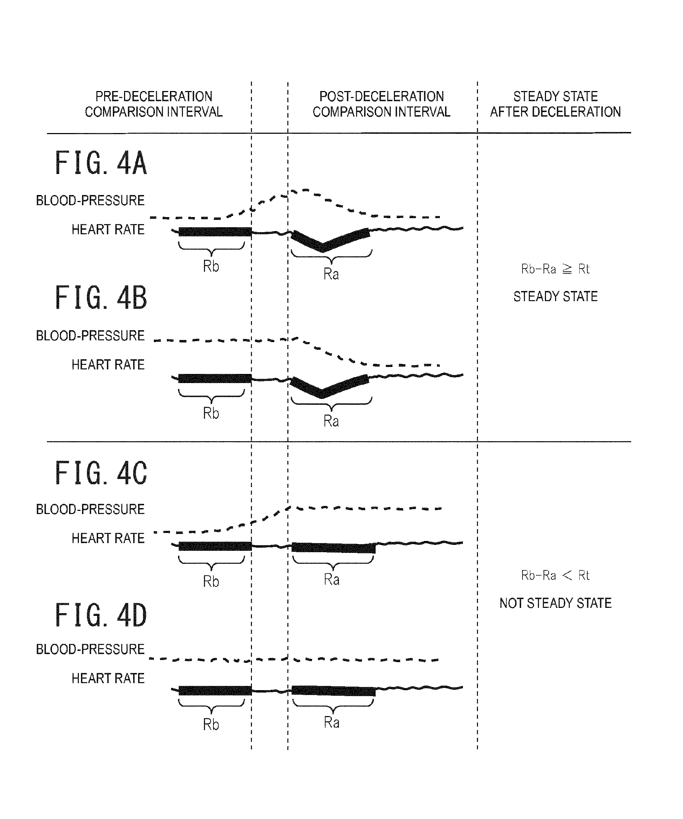

FIG. 4A to FIG. 4D are views the estimation of the steady state.

First, as illustrated in FIG. 4A and FIG. 4B, the driver's state estimation unit 24 is configured to estimate that the driver after the deceleration is in the steady state when the difference ΔR (=Rb-Ra) between the pre-deceleration heart rate Rb and the post-deceleration heart rate Ra is equal to or larger than a predetermined threshold value Rt. Herein, FIG. 4A illustrates a case where the blood pressure which has risen due to the deceleration behavior decreases after the deceleration. FIG. 4B illustrates a case where the blood pressure which has risen before the deceleration behavior decreases after the deceleration.

On the other hand, as illustrated in FIG. 4C and FIG. 4D, the driver's state estimation unit 24 is configured to estimate that the driver after the deceleration is not in the steady state when the difference ΔR (=Rb-Ra) between the pre-deceleration heart rate Rb and the post-deceleration heart rate Ra is less than the predetermined threshold value Rt. Herein, FIG. 4C illustrates a case where the blood pressure which has risen due to the deceleration behavior is kept after the deceleration. FIG. 4D illustrates a case where the blood pressure does not change before the deceleration behavior and after the deceleration.

It is noted that the one threshold value Rt is set in the above description, and two phases in which the driver after the deceleration is in the steady state or not is estimated depending on whether or not the difference ΔR between the pre-deceleration heart rate Rb and the post-deceleration heart rate Ra is larger than the threshold value Rt, the present discloser is not limited thereto. That is, three or more phases of the steady state of the driver after the deceleration, for example, "steady state", "a bit steady state", "not steady state", and the like, may be estimated by setting plural threshold values and depending on the range where the difference ΔR falls into.

What is described above is the descriptions of the driver's state estimation processing based on the block diagram in FIG. 2.

Next, the driver' s state estimation processing will be described based on a flowchart.

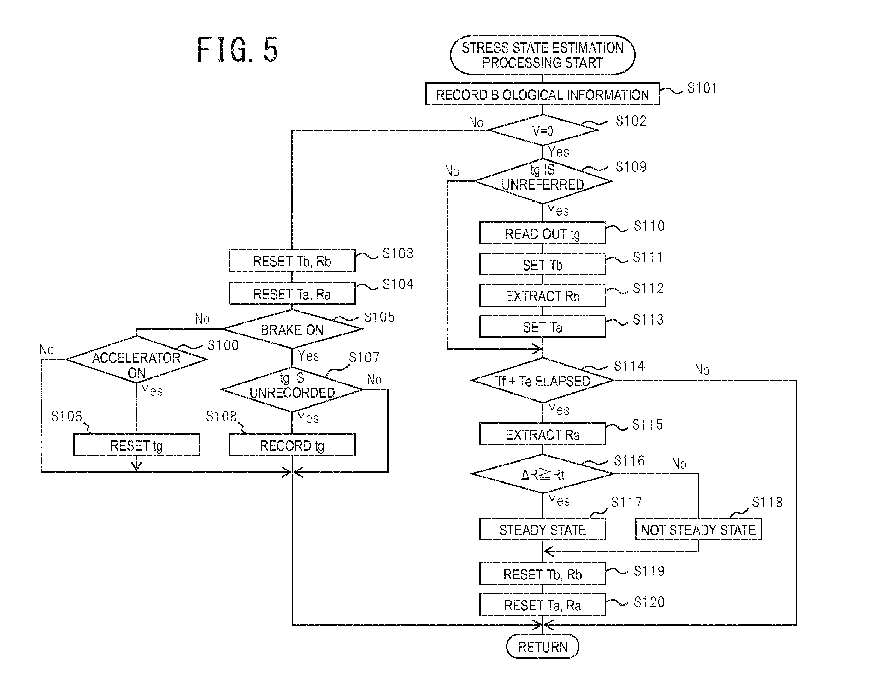

FIG. 5 is the flowchart illustrating the driver's state estimation processing.

First, in the step S101 corresponding to a processing in the biological information record unit 21, the heart rate R is calculated on the basis of the pulse wave detected by the pulse wave sensor 11 and the electrocardiographic waveform detected by the electrocardiographic monitor 12 and the heart rate R is recorded as the biological information.

In the step S102, it is determined whether or not the vehicle speed V is zero. Now, when the vehicle speed V is faster than zero, it is determined that the vehicle is in the travelling state, and the processing proceeds to the step S103. On the other hand, when the vehicle speed V is zero, it is determined that the vehicle is in a stopped state, and the processing proceeds to the step S109.

In the step S103, the pre-deceleration comparison period Tb and the pre-deceleration heart rate Rb which are set by the biological information extraction unit 23 are reset.

In the following step S104, the post-deceleration comparison period Ta and the post-deceleration heart rate Ra which are set by the biological information extraction unit 23 are reset.

In the following step S105, it is determined whether or not the break including the engine brake is ON. Now, when the break is OFF, it is determined that the deceleration operation is not performed and the processing proceeds to the step S100. On the other hand, when the brake is ON, it is determined that the deceleration operation is performed and the processing proceeds to the step S107. It is noted that the driver may continuously switch the braking operation between ON and OFF, such as in a case of the intermittent brake operation or the like. Thus, when the brake switch 14 switches from OFF to ON, the brake switch 14 may be considered as being remaining in the ON state until a predetermined time elapses.

In the step S100, it is determined whether or not the accelerator is ON. Now, when the accelerator is ON, it is determined that the acceleration operation is performed and the processing proceeds to the step S106. On the other hand, the accelerator is OFF, it is determined that the acceleration operation is not performed and the processing returns to a predetermined main program.

In the step S106 corresponding to a processing in the deceleration start time detection unit 22, the deceleration start time tg is reset and the processing returns to the predetermined main program.

In step S107, it is determined whether or not the deceleration start time tg is unrecorded. Now, when the deceleration start time tg is unrecorded, it is determined that it is just after starting the brake operation, and the processing proceeds to the step S108. On the other hand, when the deceleration start time tg is recorded, it is determined that it is not just after starting the brake operation, and the processing returns to the predetermined main program.

In the step S108, the deceleration start time tg when the vehicle in the travelling state starts to decelerate is recorded, and then the processing returns to the predetermined main program.

In the step S109, it is determined whether or not the deceleration start time tg is unreferred to. Now, when the deceleration start time tg is unreferred to, it is determined that it is just after the vehicle stops, the processing proceeds to the step S110. On the other hand, when the deceleration start time tg is referred to, it is determined that it is not just after the vehicle stops, the processing proceeds to the step S114.

In the step S110 corresponding to a processing in the deceleration start time detection unit 22, the deceleration start time tg when the vehicle in the travelling state starts to decelerate is read out (referred to).

In the following step S111 corresponding to a processing in the biological information extraction unit 23 and the pre-deceleration comparison period Tb is set. That is, the pre-deceleration reference start time tb1 prior to the the deceleration start time tg by the predetermined time Tr is set is set and the deceleration start time tg is set as the pre-deceleration reference end time tb2. And the period from the pre-deceleration reference start time tb1 to the pre-deceleration reference end time tb2 is set as the pre-deceleration comparison period Tb.

In the following step S112 corresponding to the processing in the biological information extraction unit 23, the data group of the heart rates R recorded within the pre-deceleration comparison period Tb is referred to, and the average of the heart rates R is extracted as the pre-deceleration heart rate Rb.

In the following step S113 corresponding to the processing in the biological information extraction unit 23, the post-deceleration comparison period Ta is set. That is, the required time Tf from the deceleration start time tg until the biological information of the driver becomes stable is set, and the post-deceleration reference start time ta1 when the predetermined time Tf elapses from the deceleration start time tg is set. And the post-deceleration reference end time ta2 when the predetermined time Te elapses from the post-deceleration reference start time ta1 is set. Then, the post-deceleration comparison period Ta from the post-deceleration reference start time ta1 to the post-deceleration reference end time ta2 is set.

In the following step S114, it is determined that whether or not the time (Tf + Te) has elapsed from the deceleration start time tg. The time (Tf + Te) corresponds to the time from when the deceleration starts and the heart rate R decreases until the heart rate R returns to the same status as before the start of deceleration. Herein, when the time (Tf + Te) has not elapsed, it is determined that the post-deceleration comparison period Ta has not elapsed, and thus the post-deceleration heart rate Ra can not be extracted. And the processing returns to the predetermined main program. On the other hand, when the time (Tf + Te) has elapsed, it is determined that the post-deceleration comparison period Ta has elapsed, and thus the post-deceleration heart rate Ra can be extracted. And the processing proceeds to the step S115.

In the step S115 corresponding to a processing in the biological information extraction unit 23, the data group of the heart rates R recorded within the post-deceleration comparison period Ta is referred to and the average of the heart rates R is extracted as the post-deceleration heart rate Ra.

In the following step S116 corresponding to a processing in the driver's state estimation unit 24, it is determined whether or not the difference $\Delta R$ between the pre-deceleration heart rate Rb and the post-deceleration heart rate Ra is equal to or larger than the predetermined threshold value Rt. Now, when the difference $\Delta R$ is equal to or larger than the predetermined threshold value Rt, it is estimated that the driver after the deceleration is in the steady state, and the processing proceeds to the step S117. On the other hand, when the difference $\Delta R$ is smaller than the predetermined threshold value Rt, it is estimated that the driver after the deceleration is not in the steady state, and the processing proceeds to the step S118.

In the step S117 corresponding to the processing in the driver's state estimation unit 24, the estimation result that the driver after the deceleration is in the steady state is recorded as a history, for example in a predetermined drive recorder, and then the processing proceeds to the step S119.

In the step S118 corresponding to the processing in the driver's state estimation unit 24, the estimation result that the driver after the deceleration is not in the steady state is recorded as a history, for example in the predetermined drive recorder, and then the processing proceeds to the step S119.

In the step S119, the pre-deceleration comparison period Tb and the pre-deceleration heart rate Rb which are set by the biological information extraction unit 23 are reset, and then the processing returns to the predetermined main program.

The following step S120, the post-deceleration comparison period Ta and the post-deceleration heart rate Ra which are set by the biological information extraction unit 23 are reset, and then the processing returns to the predetermined main program.

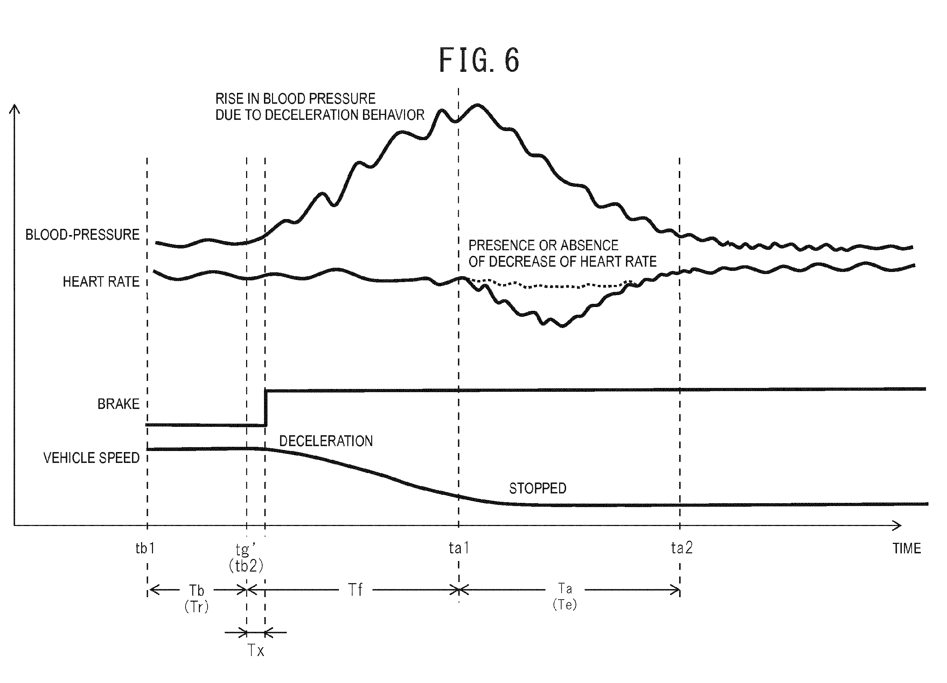

What is described above is the descriptions of the driver's state estimation processing based on the flowchart in FIG. 6.

(Operation)

Next, the operation of the first embodiment will be described.

With regard to the blood-pressure as one example of the biological information, a normal value can be obtained only when a measuring object person is in the steady state. For example, it is known that the blood-pressure becomes higher due to exercise, meals, an excited state, a nervous state, smoking, a coldness, or the like, and contrarily, the blood-pressure becomes lower due to a warmness, a steady state, relaxed state, or the like. Among these factors, the factors other than the mental factors can be self-controlled. However, the mental factors may not be sufficiently self-controlled.

Furthermore, some people experience a rise in blood pressure only by wearing a sphygmomanometer cuff. Thus, multiple measurements may be needed for measuring a blood-pressure value in the real steady state. Therefore, a user has to reduce the various fluctuating factor by taking a break for a few minutes before measuring the blood-pressure to secure the accuracy of the measured values. And the user also has to consider that the various fluctuating factor may be affected by the physical exercise or the emotional ups and downs of the user. Thus, in measuring the biological information, the measurement of the biological information in the steady state with high accuracy is difficult and an important problem.

Therefore, in the present embodiment, focusing on the tendency of the heart rate R of the driver to be stabilized uniformity after the change of the heart rate R of the driver due to the deceleration of the vehicle, it is estimated whether or not the driver after the deceleration is in the steady state by comparing the pre-deceleration heart rate Rb and the post-deceleration heart rate Ra.

First, the heart rate R as the vital sign of the circulatory system of the driver is calculated on the basis of the pulse wave and the electrocardiographic waveform of the driver and recorded with the time information (the step S101). In the present embodiment, focusing on the tendency of the heart rate R of the driver to be stabilized uniformity after the change of the heart rate R of the driver due to the deceleration of the vehicle, by observing how the heart rate R of the driver changes before and after the deceleration start time tg before the vehicle stops, the steady state of the driver after the deceleration is estimated.

First, the time when the brake switches from OFF to ON ("Yes" in the determination in the step S105) in the state where the vehicle speed V is faster than zero ("No" in the determination in the step s102) is recorded as the deceleration start time tg (the step S108) . Then, the recorded deceleration start time tg is read out (the step S110) at a vehicle stop time ts when the vehicle is stopped by the deceleration operation ("Yes" in the determination in the step S102).

Then, as illustrated in FIG. 3, the pre-deceleration reference start time tb1 and the pre-deceleration reference end time tb2 are set in accordance with the deceleration start time tg and the predetermined time Tr, and the period from the pre-deceleration reference start time tb1 to the pre-deceleration reference end time tb2 is set as the pre-deceleration comparison period Tb (the step S111). Then, the heart rates R within the pre-deceleration comparison period Tb are referred to and the average of the data group of the heart rates R is extracted as the pre-deceleration heart rate Rb (the step S112). In the pre-deceleration comparison period Tb, both of the blood-pressure value and the heart rate are in stable state.

Furthermore, as illustrated in FIG. 4A to FIG. 4D, the post-deceleration reference start time ta1 and the post-deceleration reference end time ta2 are set in accordance with the deceleration start time tg, the predetermined time Tf and the predetermined time te, and the period from the post-deceleration reference start time ta1 to the post-deceleration reference end time ta2 is set as the post-deceleration comparison period Ta (the step S113). In this case, in the period from the deceleration start time tg to the vehicle stop time ts, the heart rate does not change significantly generally, however the blood-pressure has a tendency to rise due to the deceleration operation. Then, when the vehicle is stopped completely, the blood-pressure starts to drop from the state where the blood-pressure has risen, and returns to the stable state. On the other hand, the heart rate R also drops temporarily, and starts to rise again, and then returns to the stable state.

Then, the processing waits from the deceleration start time tg until the time (Tf+Te) elapses ("No" in the determination of the step S114), that is, until the data collection of the heart rates R within the post-deceleration comparison period Ta is completed. Then, when the time (Tf+Te) elapses ("Yes" in the determination of the step S114), the heart rates R within the post-deceleration comparison period Ta are referred to, and the average of the data group of the heart rates R is extracted as the post-deceleration heart rate Ra (the step S115). In the post-deceleration comparison period Ta, both of the heart rate R and the heart rate are in the stable state.

Then, as illustrated in FIG. 4A and FIG. 4B, it is estimated that the driver after the deceleration is in the steady state (the step S117) when the difference ΔR between the pre-deceleration heart rate Rb and the post-deceleration heart rate Ra is equal to or larger than the predetermined threshold value Rt ("Yes" in the determination in the step S116). On the other hand, as illustrated in FIG. 4C and FIG 4D, it is estimated that the driver after the deceleration is not in the steady state (the step S118) when the difference ΔR is smaller than the predetermined threshold value Rt. In this way, by comparing the pre-deceleration heart rate Rb and the post-deceleration heart rate Ra, it is possible to estimate whether or not the driver after the deceleration is in the steady state with high accuracy. Furthermore, since it is estimated whether or not the driver is in the steady state by using the magnitude of the difference ΔR between the pre-deceleration heart rate Rb and the post-deceleration heart rate Ra, it is easy to estimate whether or not the driver is in the steady state.

Furthermore, by setting the pre-deceleration comparison period Tb on the basis of the deceleration start time tg and the predetermined time Tr, and referring to the data group of the heart rates R recorded within the pre-deceleration comparison period Tb, it is possible to easily extract the pre-deceleration heart rate Rb with high reliability, which can be considered as the heart rate in the steady state before the deceleration. Furthermore, by extracting the average value of the data group of the heart rates R recorded within the pre-deceleration comparison period Tb as the pre-deceleration heart rate Rb, it is possible to easily extract the statistically representative value averaging the dispersion in the data observed within the pre-deceleration comparison period Tb.

Furthermore, by setting the post-deceleration comparison period Ta depending on the deceleration start time tg, the predetermined time Tf and the predetermined time Te, and referring to the data group of the heart rates R recorded within the post-deceleration comparison period Ta, it is possible to easily extract the post-deceleration heart rate Ra with high reliability, which generally drops after the deceleration. Furthermore, by extracting the average value of the data group of the heart rates R recorded within the post-deceleration comparison period Ta as the post-deceleration heart rate Ra, it is possible to easily extract the statistically representative value averaging the dispersion in the data observed within the post-deceleration comparison period Ta.

(Application Example 1)

In the present embodiment, the time when the brake is switched to ON by the driver as the deceleration start time tg, the deceleration start time tg' may be detected by adding the reaction time Tx from the time when the driver recognizes that the vehicle needs to stop until a time when the driver starts the deceleration operation.

First, the reaction time Tx from the time when the driver recognizes that the vehicle needs to stop until the time when the driver starts the deceleration operation is preset. The reaction time is obtained by summing a reflex time from recognizing that the vehicle needs to stop until the foot moves (0.4 to 0.5 sec, for example), and a retreading time from the accelerator pedal to the brake pedal (0.2 sec, for example), and a pedaling time from start of pedaling the brake pedal until the brake switch 14 switches to OFF (0.1 to 0.3 sec, for example). Thus, Tx is about 0.7 to 1.0 sec.

FIG. 6 is a time chart illustrating the reaction time Tx and the deceleration start time tg.

The deceleration start time detection unit 22 is configured to detect the time prior to the time tg when the brake switch 14 switches to ON by the reaction time Tx as the deceleration start time tg' (tg-Tx=tg').

The biological information extraction unit 23 is configured to set the time prior to the deceleration start time tg' by the predetermined time Tr is set as the pre-deceleration reference start time tb1 (tg'-Tr=tb1). Furthermore, the time when a predetermined time Tf elapses from the deceleration start time tg' is set as the post-deceleration reference start time ta1 (tg'+Tf=ta1).

Next, the operation of the above-mentioned application example will be described.

There is a slight time difference (loss of time) associated with recognition, determination and action between the time when the driver recognizes that the vehicle needs to stop and the time when the brake switch 14 switches to ON. Therefore, when the post-deceleration comparison period Ta is set on the basis of the predetermined times Tf and Te with reference to the deceleration start time tg when the brake switch 14 switches to ON, the heart rate R just before the post-deceleration reference start time ta1 may have already started to drop. In this case, the post-deceleration heart rate Ra is extracted in a state where the heart rate R has already drops outside the post-deceleration comparison period Ta, the estimate accuracy of the steady state may be subject to the influence.

Therefore, in order to improve the estimate accuracy of the steady state, it is necessary to correctly set the pre-deceleration comparison period Tb and the post-deceleration comparison period Ta, and thus it is needed to accurately detect the deceleration start time tg'.

Therefore, in this application example, the reaction time Tx from the time when the driver recognizes that the vehicle needs to stop until the time when the driver starts the deceleration operation is set, the time prior to the time tg when the brake switch 14 switches to ON by the reaction time Tx is detected as the deceleration start time tg'. In this way, the deceleration start time tg' approximates the time when the driver recognizes that the vehicle needs to stop. Therefore, it is possible to correctly set the pre-deceleration comparison period Tb and the post-deceleration comparison period Ta, and thus it is possible to improve the estimate accuracy of the steady state.

In the above description, the processing in the step S101 in the biological information record unit 21 corresponds to the "biological information record unit", and the processing in the steps S108 and S110 in the deceleration start time detection unit 22 corresponds to the "deceleration start time detection unit". Furthermore, the processing in the steps S110 to S115 in the biological information extraction unit 23 corresponds to the "biological information extraction unit", and the processing in the steps S116 to S118 in the driver's state estimation unit 24 corresponds to the "driver's state estimation unit".

(Effects)

Next, effects of the main part of the first embodiment will be described.

(1) In the driver's state estimation apparatus according to the present embodiment, the biological information of the driver is recorded by the processing in the biological information record unit 21, and the deceleration start time tg when the vehicle in the travelling state starts to decelerate and stops is detected by the processing in the deceleration start time detection unit 22. Furthermore, the heart rates R within the predetermined period recorded before the deceleration start time tg by the biological information record unit 21 are referred to, and the pre-deceleration heart rate Rb which is the representative value of the referred heart rates R is extracted by the processing of the biological information extraction unit 23. Furthermore, the heart rates R within the predetermined period recorded after the deceleration start time tg by the biological information record unit 21 are referred to, and the post-deceleration heart rate Ra which is the representative value of the referred heart rates R is extracted. Then, the pre-deceleration heart rate Rb and the post-deceleration heart rate Ra extracted by the biological information extraction unit 23 are compared with each other to estimate whether or not the driver after the deceleration is in the steady state by the processing in the driver's state estimation unit 24.

In this way, the pre-deceleration heart rate Rb in the stable state before the deceleration start time tg and the post-deceleration heart rate Ra which may change after the deceleration start time tg are compared with each other to estimate the steady state of the driver, and thus it is possible to improve the estimate accuracy. That is, focusing on the tendency of the heart rate R of the driver to be stabilized uniformity after the heart rate R of the driver drops due to the deceleration of the vehicle, the pre-deceleration heart rate Rb which can be considered as a heart rate in the stable state and the post-deceleration heart rate Ra which may be change are compared with each other. Thus, it is possible to estimate with high accuracy whether or not the driver after the deceleration is in the steady state.

(2) In the driver's state estimation apparatus according to the present embodiment, when the difference ΔR between the pre-deceleration heart rate Rb and the post-deceleration heart rate Ra is equal to or larger than the predetermined threshold value Rt, it is estimated that the driver after the deceleration is in the steady state by the processing in the driver's state estimation unit 24.

In this way, it is estimated whether or not the driver after the deceleration is in the steady state by using the magnitude of the difference ΔR between the pre-deceleration heart rate Rb and the post-deceleration heart rate Ra, thus it is easy to estimate whether or not the driver is in the steady state.

(3) In the driver's state estimation apparatus according to the present embodiment, the time prior to the deceleration start time tg by the predetermined time Tr is set as the pre-deceleration reference start time tb1 and the deceleration start time tg is set as the pre-deceleration reference end time tb2, by the processing in the biological information extraction unit 23. Then, the heart rates R from the pre-deceleration reference start time tb1 to the pre-deceleration reference end time tb2 from the heart rates R recorded by the biological information record unit 21 are referred to, and the pre-deceleration heart rate Rb which is the presentative value of the referred heart rates R is extracted.

In this way, the data group of the heart rates R recorded within the pre-deceleration comparison period Tb is referred to. Thus, it is possible to easily extract the pre-deceleration heart rate Rb with high reliability, which can be considered as the heart rate in the steady state before the deceleration.

(4) In the driver's state estimation apparatus according to the present embodiment, the time when the predetermined time Tf elapses from the deceleration start time tg is set as the post-deceleration reference start time ta1 and the time when the predetermined time Te elapses from the post-deceleration reference start time ta1 is set as the post-deceleration reference end time ta2, by the processing in the biological information extraction unit 23. Then, the heart rates R from the post-deceleration reference start time ta1 to the post-deceleration reference end time ta2 from the heart rates R recorded by the biological information record unit 21 are referred to, and the post-deceleration heart rate Ra which is the presentative value of the referred heart rates R is extracted.

In this way, the data group of the heart rates R recorded within the post-deceleration comparison period Ta is referred to. Thus, it is possible to easily extract the post-deceleration heart rate Ra with high reliability, which can be considered as the heart rate in the steady state after the vehicle stops.

(5) In the driver's state estimation apparatus according to the present embodiment, the heart rates R within the predetermined period recorded before the deceleration start time tg by the biological information record unit 21 are referred to, and the average value of the referred heart rates R is extracted as the pre-deceleration heart rate Rb, by the processing of the biological information extraction unit 23. Furthermore, the heart rates R within the predetermined period recorded after the deceleration start time tg by the biological information record unit 21 are referred to, and the average value of the referred heart rates R is extracted as the post-deceleration heart rate Ra.

In this way, the average value of the data group of the heart rates R recorded within the pre-deceleration comparison period Tb is extracted as the pre-deceleration heart rate Rb. Thus, it is possible to easily extract the statistically representative value averaging the dispersion in the data observed within the pre-deceleration comparison period Tb. Furthermore, the average value of the data group of the heart rates R recorded within the post-deceleration comparison period Ta is extracted as the post-deceleration heart rate Ra. Thus, it is possible to easily extract the statistically representative value averaging the dispersion in the data observed within the post-deceleration comparison period Ta.

(6) In the driver's state estimation apparatus according to the present embodiment, the heart rate R of the driver is recorded as the biological information, by the processing in the biological information record unit 21.

In this way, the heart rate R which is affected by the autonomic nerve is recorded. Thus, it is possible to estimate with high accuracy whether or not the driver after the deceleration is in the steady state.

(7) In the driver's state estimation apparatus according to the present embodiment, the reaction time Tx from the time when the driver recognizes that the vehicle needs to stop until the time when the driver starts the deceleration operation is preset by the processing of the deceleration start time detection unit 22. Then, the time prior to the time tg when the driver starts the deceleration operation by the reaction time Tx is detected as the deceleration start time tg'. In this way, the deceleration start time tg' is detected by adding the reaction time Tx from the time when the driver recognizes that the vehicle needs to stop until the time when the driver starts the deceleration operation. Therefore, it is possible to correctly set the post-deceleration comparison period Ta, and thus it is possible to improve the estimate accuracy of the steady state.

(8) In the driver's state estimation method according to the present embodiment, the heart rate R is recorded, and the deceleration start time tg when the vehicle in the travelling state starts to decelerate and stops is detected. Then, the heart rates R within the predetermined period recorded before the deceleration start time tg are referred to, and the pre-deceleration heart rate Rb which is the representative value of the referred heart rates R is extracted. And, the heart rates R within the predetermined period recorded after the deceleration start time tg are referred to, and the post-deceleration heart rate Ra which is the representative value of the referred heart rates R is extracted. Then, the pre-deceleration heart rate Rb and the post-deceleration heart rate Ra are compared with each other to estimate whether or not the driver after the deceleration is in the steady state.

In this way, the pre-deceleration heart rate Rb in the stable state before the deceleration start time tg and the post-deceleration heart rate Ra which may change after the deceleration start time tg are compared with each other to estimate the steady state of the driver, and thus it is possible to improve the estimate accuracy. That is, focusing on the tendency of the heart rate R of the driver to be stabilized uniformity after the heart rate R of the driver drops due to the deceleration of the vehicle, the pre-deceleration heart rate Rb which can be considered as a heart rate in the stable state and the post-deceleration heart rate Ra which may change are compared with each other. Thus, it is possible to estimate with high accuracy whether or not the driver after the deceleration is in the steady state.

(Second Embodiment)

(Configuration)

The present embodiment estimates whether or not the driver after the deceleration is in the steady state with high accuracy, and acquires driver's information when the driver is in the steady state.

Herein, only the differences from the above-mentioned first embodiment will be described, and descriptions on the identical elements are omitted.

Hereinafter, the driver's state estimation processing performed by the controller 17 will be described.

First, the driver's state estimation processing of the second embodiment will be described based on a block diagram.

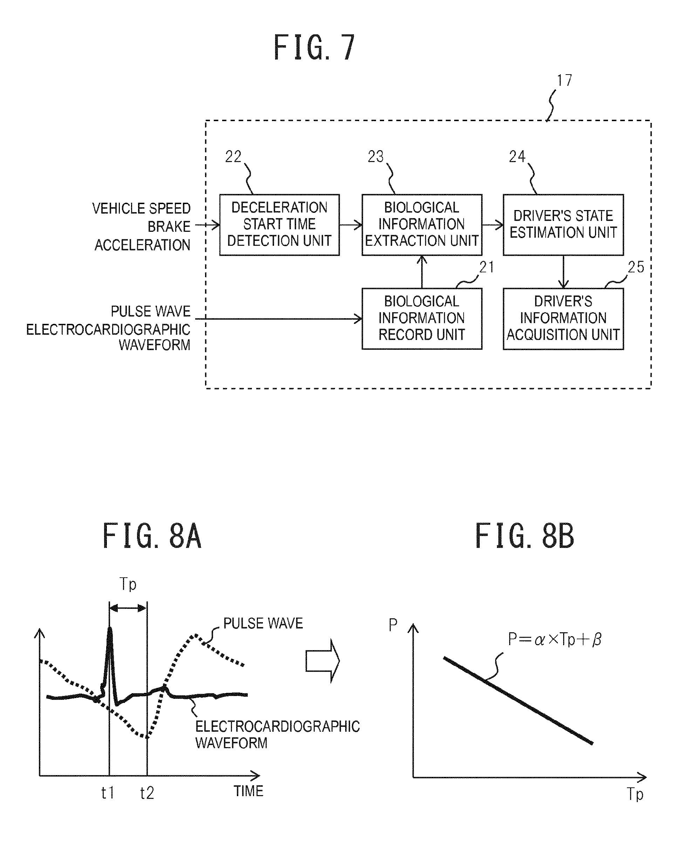

FIG. 7 is the block diagram illustrating the driver's state estimation processing of the second embodiment.

In the second embodiment, the above-mentioned processing in the biological information record unit 21, the biological information extraction unit 23, and the driver's state estimation unit 24 are modified, and a driver's information acquisition unit 25 is added. With regard to the other processing, the second embodiment is the same as the above-mentioned first embodiment, thus, descriptions thereof are omitted.

The biological information record unit 21 is configured to calculate the blood-pressure value P in addition to the heart rate R, and to record the blood-pressure value P as the biological information, too. The biological information record unit 21 is configured to accumulate the blood-pressure values P with time information in a nonvolatile memory and manage them.

In the present embodiment, the blood-pressure value P is calculated based on a pulse transmission time Tp, by utilizing a negative correlation between the blood-pressure value P and the pulse transmission time Tp which is a time from the pumping of the heart until the pulse arrives at the periphery.

FIG. 8A and FIG. 8B are views illustrating a calculation method of the blood-pressure value P.

FIG. 8A illustrates the pulse transmission time Tp depending on the pulse wave and the electrocardiographic waveform. FIG. 8B illustrates the blood-pressure value P depending on the pulse transmission time Tp. Furthermore, in FIG. 8A, the time t1 when a peak value of electrocardiographic waveform appears is the time of the pumping of the heart. The time t2 when a peak value of the pulse wave appears is the time when the pulse arrives at the periphery. The difference (t2-t1) between the peak time t2 of the pulse wave and the peak time t1 of the electrocardiographic waveform is the pulse transmission time Tp. Furthermore, as illustrated in FIG. 8B, the pulse transmission time Tp and blood-pressure value P have a relationship of $P=\alpha \cdot Tp+\beta$. The blood-pressure value P is calculated on the basis of the pulse transmission time Tp in accordance with the relationship expression.

What is described above is the record processing of the blood-pressure value P by the biological information record unit 21.

The biological information extraction unit 23 is configured to refer the blood-pressure values P within a predetermined post-deceleration stability period Ts recorded after the deceleration start time tg by the biological information record unit 21, and extract an average change rate p and a standard deviation $\sigma$ of the referred pressure values P.

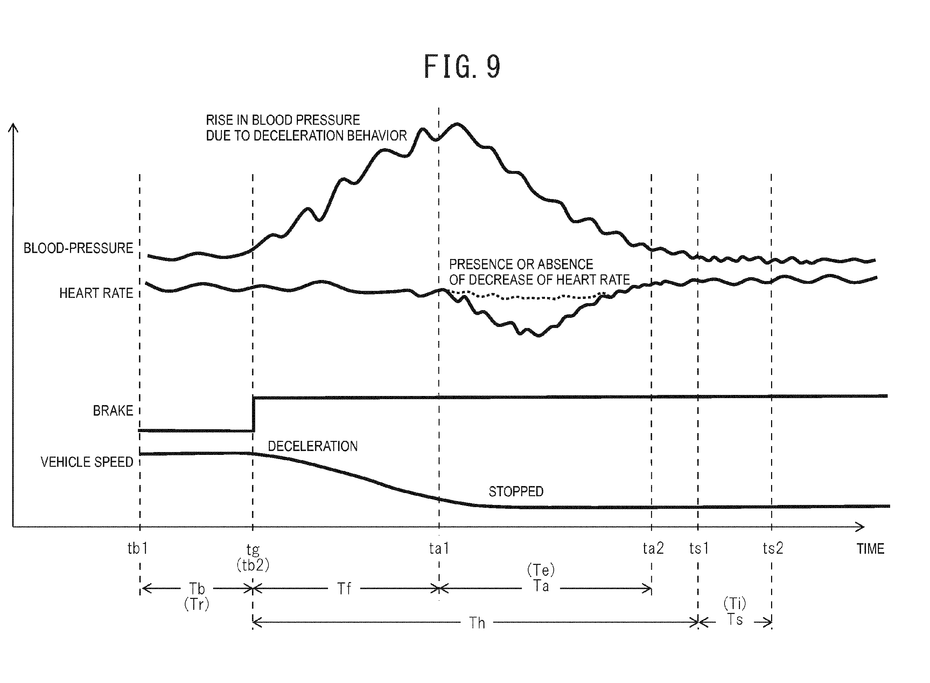

FIG. 9 is a time chart illustrating the post-deceleration stability period Ts.

A time when a predetermined time Th elapses from the deceleration start time tg is set as a stability period reference start time ts1 (tg+Th=ts1), and a time when the predetermined time Ti elapses from the stability period reference start time ts1 is set as a stability period reference end time ts2 (ts1+Ti=ts2). Herein, the time Th corresponds to a time from the start of the deceleration until the heart rate R which has changed starts to stabilize again. Then, a period from the stability period reference start time ts1 to the stability period reference end time ts2 is set as the post-deceleration stability period Ts (ts2-ts1=Ts=Ti).

Then, the data group of the pressure values P recorded within the post-deceleration stability period Ts is referred to, and the average change rate p and the standard deviation $\sigma$ of the pressure values P are extracted.

What is described above is the extraction processing of the average change rate p and the standard deviation $\sigma$ by the biological information extraction unit 23.

The driver's state estimation unit 24 is configured to estimate that the driver after the deceleration is in the steady state when the difference $\Delta R$ between the pre-deceleration heart rate Rb and the post-deceleration heart rate Ra is equal to or larger than the predetermined threshold Rt and when the average change rate p is smaller than the predetermined threshold value pt. Or, the driver's state estimation unit 24 is configured to estimate that the driver after the deceleration is in the steady state when the difference $\Delta R$ between the pre-deceleration heart rate Rb and the post-deceleration heart rate Ra is equal to or larger than the predetermined threshold Rt and when the standard deviation $\sigma$ is smaller than the predetermined threshold value $\sigma t$.

The driver's information acquisition unit 25 is configured to acquire at least one of blood-pressure information, electrocardiogram information, heart rate information, respiratory information, pulse wave information, electrodermal activity information, body temperature information, facial expression information, and utterance information of the driver as the driver's information when it is estimated by the driver's state estimation unit 24 that the driver after the deceleration is in the steady state.

What is described above is the descriptions of the driver's state estimation processing based on the block diagram in FIG. 7.

Next, the driver's state estimation processing of the second embodiment will be described based on a flowchart.

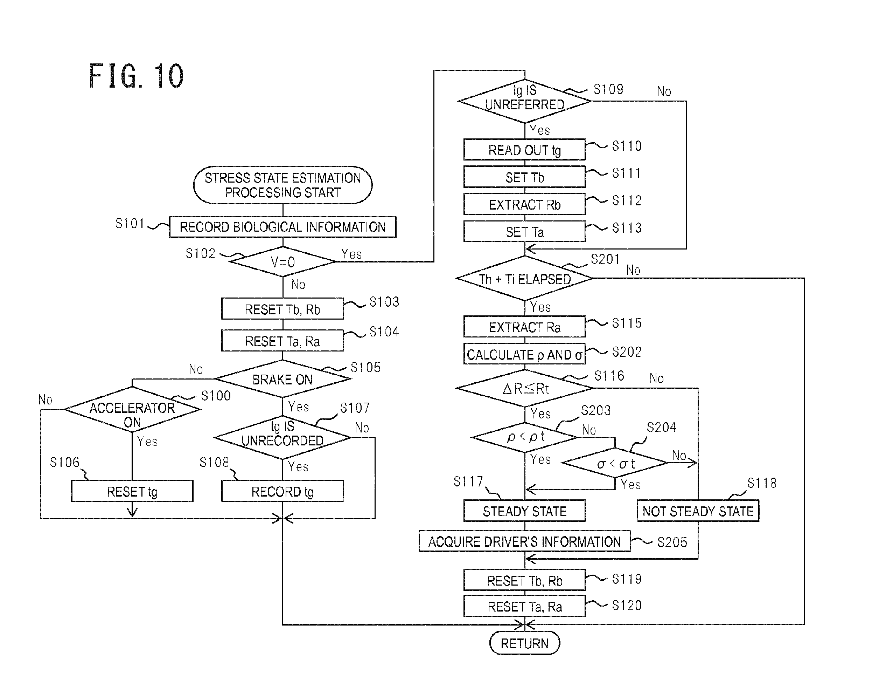

FIG. 10 is the flowchart illustrating the driver's state estimation processing of the second embodiment.

Herein, the processing of the above-mentioned step S114 is changed to new processing of the step S201, and new processing of the steps S202 to S205 is added. With respect to the processing of the other steps S101 to S120, and S100, the second embodiment is the same as the above-mentioned first embodiment, thus, descriptions thereof are omitted.

In the step S201, it is determined whether or not the time (Th+Ti) has elapsed from the deceleration start time tg. The time Th corresponds to a time from the start of the deceleration until the heart rate R which has changed starts to stabilize again. Now, when the time (Th+Ti) has not elapsed yet, it is determined that the post-deceleration stability period Ts has not elapsed, and thus it is not possible to extract the average change rate p and the standard deviation $\sigma$ of the pressure values P. Then, the processing returns to the predetermined main program. On the other hand, when the time (Th+Ti) has elapsed, it is determined that it is possible to extract the average change rate p and the standard deviation $\sigma$ of the pressure values P. Then, the processing proceeds to the step S115.

In the step S202 corresponding to the processing of the biological information extraction unit 23, the blood-pressure values P within the predetermined post-deceleration stability period Ts recorded after the deceleration start time tg by the biological information record unit 21 are referred to, and the average change rate p and the standard deviation $\sigma$ of the referred pressure values P are extracted. Then, the processing proceeds to the step S116.

In the step S203 corresponding to the processing of the driver's state estimation unit 24, it is determined whether or not the average change rate p of the pressure values P is smaller than the predetermined threshold pt. Now, when the average change rate p of the pressure values P is smaller than the predetermined threshold pt, it is estimated that the driver after the deceleration is in the steady state, and then the processing proceeds to the step S117. On the other hand, when the average change rate p of the pressure values P is equal to or lager than the predetermined threshold pt, it is estimated that the driver after the deceleration may not be in the steady state, and then the processing proceeds to the step S204.

In the step S204 corresponding to the processing of the driver's state estimation unit 24, it is determined whether or not the standard deviation $\sigma$ of the pressure values P is smaller than the predetermined threshold $\sigma t$. Now, when the standard deviation $\sigma$ of the pressure values P is smaller than the predetermined threshold $\sigma t$, it is estimated that the driver after the deceleration is in the steady state, and then the processing proceeds to the step S117. On the other hand, when the standard deviation $\sigma$ of the pressure values P is equal to or lager than the predetermined threshold $\sigma t$, it is estimated that the driver after the deceleration may not be in the steady state, and then the processing proceeds to the step S118.

In the step S205 corresponding to the processing of the driver's information acquisition unit 25, at least one of the blood-pressure information, the electrocardiogram information, the heart rate information, the respiratory information, the pulse wave information, the electrodermal activity information, the body temperature information, the facial expression information, and the utterance information of the driver is acquired as the driver's information, and then the processing proceeds to the step S119.

What is described above is the descriptions of the driver's state estimation processing based on the flowchart in FIG. 10.

(Operation)

Next, the operation of the second embodiment will be described.

In the present embodiment, it is estimated whether or not the driver after the deceleration is in the steady state on the basis of not only the difference $\Delta R$ between the pre-deceleration heart rate Rb and the post-deceleration heart rate Ra but also the average change rate p and the standard deviation $\sigma$ of the pressure values P. That is, when the time (Th+Ti) has elapsed from the deceleration start time tg ("Yes" in the determination in the step S201), the average change rate p and the standard deviation $\sigma$ of the pressure values P are extracted.

Then, it is estimated that the driver after the deceleration is surely in the steady state when the difference $\Delta R$ between the pre-deceleration heart rate Rb and the post-deceleration heart rate Ra is equal to or larger than the predetermined threshold Rt ("Yes" in the determination in the step S116) and when the average change rate p of the pressure values P is smaller than the predetermined threshold value pt ("Yes" in the determination in the step S203). In this situation, even when the average change rate p of the pressure values P is equal to or larger than the predetermined threshold value pt ("No" in the determination in the step S203), when the standard deviation $\sigma$ of the pressure values P is smaller than the predetermined threshold value $\sigma t$ ("Yes" in the determination in the step S204), it is estimated that the driver after the deceleration is surely in the steady state.

By considering not only the difference $\Delta R$ between the pre-deceleration heart rate Rb and the post-deceleration heart rate Ra but also the average change rate p and the standard deviation $\sigma$ of the pressure values P in this way, it is possible to estimate more accurately whether or not the driver after the deceleration is in the steady state.

As described above, when it is estimated that the driver after the deceleration is surely in the steady state, it means that the driver is relaxed and is in normal state. Therefore, the information on the driver at the time should be acquired. For example, the information on the driver to be acquired may be the blood-pressure information, the electrocardiogram information, the heart rate information, the respiratory information, the pulse wave information, the electrodermal activity information, the body temperature information, the facial expression information, and the utterance information of the driver. By acquiring this information, it is possible to know the normal state where the drive is relaxed.

In the above description, the processing in the steps S203 and S204 is included in the "driver's state estimation unit", and the processing in the step S205 in the driver's information acquisition unit 25 corresponds to the "driver's information acquisition unit".

(Effect)

Next, effects of the main part of the second embodiment will be described.

(1) In the driver's state estimation apparatus according to the present embodiment, it is estimated that the driver after the deceleration is in the steady state by the processing in the 24, when the difference $\Delta R$ between the pre-deceleration heart rate Rb and the post-deceleration heart rate Ra is larger than the predetermined threshold Rt, and the average change rate p of the blood-pressure values P from the stability period reference start time ts1 to the stability period reference end time ts2 referred from the blood-pressure values P recorded by the biological information record unit 21 is smaller than the threshold value pt.

By considering not only the difference $\Delta R$ between the pre-deceleration heart rate Rb and the post-deceleration heart rate Ra but also the average change rate p of the pressure values P in this way, it is possible to estimate more accurately whether or not the driver after the deceleration is in the steady state.

(2) In the driver's state estimation apparatus according to the present embodiment, it is estimated that the driver after the deceleration is in the steady state by the processing in the driver's state estimation unit 24, when the difference $\Delta R$ between the pre-deceleration heart rate Rb and the post-deceleration heart rate Ra is larger than the predetermined threshold Rt and the standard deviation $\sigma$ of the blood-pressure values P from the stability period reference start time ts1 to the stability period reference end time ts2 referred from the blood-pressure values P recorded by the biological information record unit 21 is smaller than the threshold value $\sigma t$,

By considering not only the difference $\Delta R$ between the pre-deceleration heart rate Rb and the post-deceleration heart rate Ra but also the standard deviation $\sigma$ of the pressure values P in this way, it is possible to estimate more accurately whether or not the driver after the deceleration is in the steady state.

(3) In the driver's state estimation apparatus according to the present embodiment, at least one of the blood-pressure information, the electrocardiogram information, the heart rate information, the respiratory information, the pulse wave information, the electrodermal activity information, the body temperature information, the facial expression information, and the utterance information of the driver is acquired as the driver's information, by the processing in the driver's information acquisition unit 25, when it is estimated by the driver's state estimation unit 24 that the driver after the deceleration is in the steady state.

By acquiring the driver's information when it is estimated that the driver after the deceleration is in the steady state, it is possible to know the normal state where the drive is relaxed.

(Third Embodiment)

(Configuration)

The present embodiment records and utilizes the driver's information acquired when it is estimated that the driver after the deceleration is in the steady state.

Herein, only the differences from the above-mentioned second embodiment will be described, and descriptions on the identical elements are omitted.

Hereinafter, the driver's state estimation processing performed by the controller 17 will be described.

First, the driver's state estimation processing of the third embodiment will be described based on a block diagram.

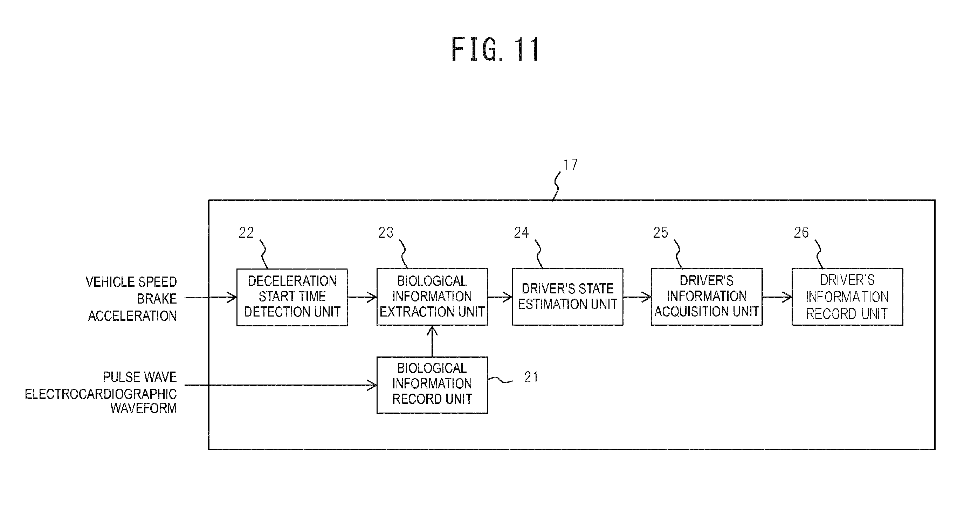

FIG. 11 is the block diagram illustrating the driver's state estimation processing of the third embodiment.

In the third embodiment, a driver's information record unit 26 is added. With regard to the other processing, the third embodiment is the same as the above-mentioned second embodiment, thus, descriptions thereof are omitted.

The driver's information record unit 26 is configured to record the driver's information acquired by the driver's information acquisition unit 25 in association with date-time information and current location information of the vehicle. Recording of this information is not limited to recording in an in-vehicle recording device, such as, the DVD-ROM drive, the hard disk drive, a flash memory drive, or the like. This information may be recorded in a personal digital assistance, a portable audio device, or the like, having a communication function

The driver's information record unit 26 is configured to further classify the driver's information on the basis of the date-time information and the current location information and to refer to the driver's information recorded in the past time, and to extract the driver's information under the same condition of date, time, traveling path, or the like, and to extract transition of the driver's information in the steady state. Furthermore, the driver's information record unit 26 is configured to extract the driver's information under the same condition of date, time, traveling path, or the like, to estimate the state of the driver in the future.

What is described above is the descriptions of the driver's state estimation processing based on the block diagram in FIG. 11.

Next, the driver's state estimation processing of the third embodiment will be described based on a flowchart.

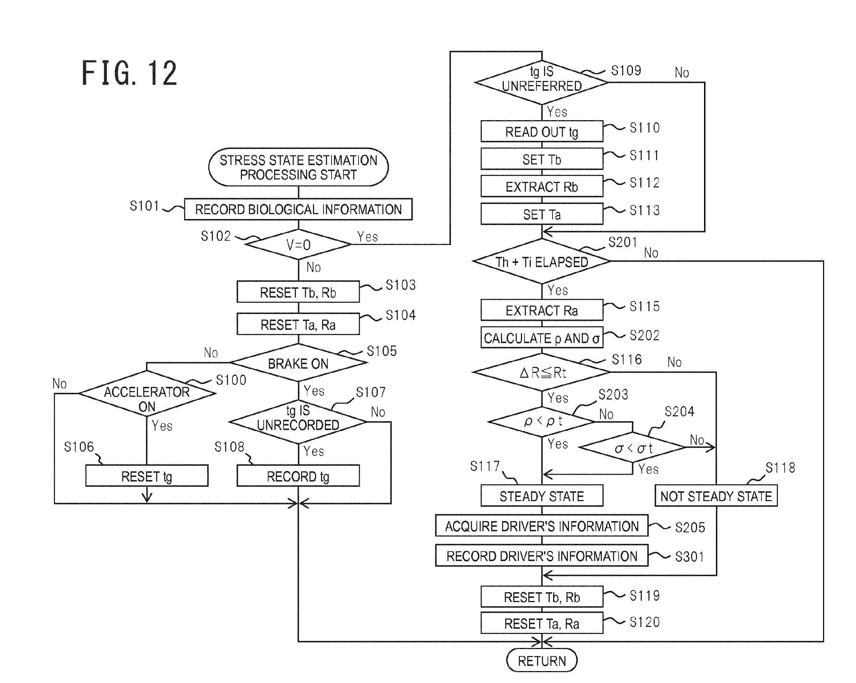

FIG. 12 is the flowchart illustrating the driver's state estimation processing of the third embodiment.

Herein, a new processing of the step S301 is added after the above-mentioned step S205. With respect to the processing of the steps S101 to S120, S100, and S201 to S205, the third embodiment is the same as the above-mentioned second embodiment, thus, descriptions thereof are omitted.

In the step 301 corresponding to the driver's information record unit 2 6, the driver's information acquired by the driver's information acquisition unit 25 is recorded in association with the date-time information and the current location information of the vehicle, then the processing proceeds to the step S119.

What is described above is the descriptions of the driver's state estimation processing based on the flowchart in FIG. 12.

(Operation)

Next, the operation of the third embodiment will be described.

Since when it is estimated that the driver after the deceleration is surely in the steady state, the driver is relaxed and is in normal state. Therefore, the information on the driver at the time is acquired and recorded in association with the date-time information and the current location information (the step S301).

This makes it possible to analyze the transition of the driver's state or to estimate how the driver's state will change in the feature, when extracting the driver's information in the past on the basis of the date-time information and the current location information.

In the above description, the processing in the step S301 in the driver's information record unit 26 corresponds to the "driver's information record unit".

(Effect)

Next, an effect of the main part of the third embodiment will be described.

(1) In the driver's state estimation apparatus according to the present embodiment, the driver's information acquired by the driver's information acquisition unit 25 is recorded in association with the date-time information and the current location information of the vehicle, by the processing in the driver's information record unit 26.

By recording the driver's information when it is estimated that the driver is surely in the steady state in this way, it is possible to analyze the transition of the driver's state or to estimate how the driver's state will change in the feature, when extracting the driver's information in the past on the basis of the date-time information and the current location information.

Priority is claimed on Japanese Patent Application No. 2012-166225 (filed on July 26, 2012), the entire content of which is incorporated by reference as a part of this application.

While the present invention has been described with reference to the definite number of embodiments, the scope of the present invention is not limited thereto and improvements and modifications of the embodiments based on the above disclosure are obvious to those skilled in the art.

Reference Signs List

11　pulse wave sensor
12　electrocardiographic monitor
13　vehicle speed sensor
14　brake switch
15　acceleration sensor
16　navigation system
17　controller

21    biological information record unit
22    deceleration start time detection unit
23    biological information extraction unit
24    driver's state estimation unit
25    driver's information acquisition unit
26    driver's information record unit

**Claims**

1.  A driver's state estimation apparatus comprising:

    a biological information record unit configured to record biological information of a driver;
    a deceleration start time detection unit configured to detect a deceleration start time when a vehicle in a travelling state starts to decelerate and stops;
    a biological information extraction unit configured to refer to the biological information within a predetermined period recorded by the biological information record unit before the deceleration start time detected by the deceleration start time detection unit to extract pre-deceleration biological information which is a representative value of the referred biological information, and to refer to the biological information within a predetermined period recorded by the biological information record unit after the deceleration start time to extract post-deceleration biological information which is a representative value of the referred biological information; and
    a driver's state estimation unit configured to compare the pre-deceleration biological information and the post-deceleration biological information extracted by the biological information extraction unit to estimate whether or not the driver after deceleration is in a steady state.

2.  The driver's state estimation apparatus according to claim 1, wherein the driver's state estimation unit is configured to estimate that the driver after the deceleration is in the steady state when a difference between the pre-deceleration biological information and the post-deceleration biological information is larger than a predetermined difference threshold.

3.  The driver's state estimation apparatus according to claim 1, wherein the biological information extraction unit is configured:

    to set a time prior to the deceleration start time by a predetermined time as a pre-deceleration reference start time;
    to set the deceleration start time as a pre-deceleration reference end time; and
    to refer the biological information from the pre-deceleration reference start time to the pre-deceleration reference end time from the biological information recorded by the biological information record unit to extract the pre-deceleration biological information which is the presentative value of the referred biological information.

4.  The driver's state estimation apparatus according to claim 1, wherein the biological information extraction unit is configured:

    to set a time when a predetermined time elapses from the deceleration start time as a post-deceleration reference start time;
    to set a time when a predetermined time elapses from the post-deceleration reference start time as a post-deceleration reference end time; and
    to refer the biological information from the post-deceleration reference start time to the post-deceleration reference end time from the biological information recorded by the biological information record unit to extract the post-deceleration biological information which is the presentative value of the referred biological information.

5.  The driver's state estimation apparatus according to claim 1, wherein the driver's state estimation unit is configured:

    to set a time when a predetermined time elapses from the deceleration start time as a stability period reference start time;
    to set a time when a predetermined time elapses from the stability period reference start time as a stability period reference end time;
    to estimate that the driver after the deceleration is in the steady state when the difference between the pre-deceleration biological information and the post-deceleration biological information is larger than the predeter-

15

mined difference threshold, and when an average change rate of the biological information from the stability period reference start time to the stability period reference end time referred from the biological information recorded by the biological information record unit is smaller than an average change ratio threshold.

6. The driver's state estimation apparatus according to claim 1, wherein the driver's state estimation unit is configured:

to set a time when a predetermined time elapses from the deceleration start time as a stability period reference start time;
to set a time when a predetermined time elapses from the stability period reference start time as a stability period reference end time;
to estimate that the driver after the deceleration is in the steady state when the difference between the pre-deceleration biological information and the post-deceleration biological information is larger than the predetermined difference threshold, and when a standard deviation of the biological information from the stability period reference start time to the stability period reference end time referred from the biological information recorded by the biological information record unit is smaller than the standard deviation threshold.

7. The driver's state estimation apparatus according to claim 1, wherein the biological information extraction unit is configured to refer the biological information within the predetermined period recorded before the deceleration start time by the biological information record unit to extract an average value of the referred biological information as the pre-deceleration biological information, and to refer the biological information within the predetermined period recorded after the deceleration start time by the biological information record unit to extract an average value of the referred biological information as the post-deceleration biological information.

8. The driver's state estimation apparatus according to claim 1, wherein the biological information record unit is configured to record a heart rate of the driver as the biological information.

9. The driver's state estimation apparatus according to claim 1, wherein further comprising a driver's information acquisition unit configured to acquire at least one of blood-pressure information, electrocardiogram information, heart rate information, respiratory information, pulse wave information, electrodermal activity information, body temperature information, facial expression information, and utterance information of the driver as driver's information, when it is estimated that the driver after the deceleration is in the steady state by the driver's state estimation unit.

10. The driver's state estimation apparatus according to claim 9, wherein further comprising a driver's information record unit configured to record the driver's information acquired by the driver's information acquisition unit in association with date-time information and current location information of the vehicle.

11. The driver's state estimation apparatus according to claim 1, wherein the deceleration start time detection unit is configured to preset a reaction time from a time when the driver recognizes that the vehicle needs to stop until a time when the driver starts a deceleration operation, and to detect a time prior to the time when the driver starts the deceleration operation by the reaction time is detected as the deceleration start time.

12. The driver's state estimation method comprising:

recording biological information of a driver;
detecting a deceleration start time when a vehicle in a travelling state starts to decelerate and stops;
referring to the biological information within a predetermined period recorded before the deceleration start time to extract pre-deceleration biological information which is a representative value of the referred biological information, and referring to the biological information within a predetermined period after the deceleration start time to extract post-deceleration biological information which is a representative value of the referred biological information; and
comparing the pre-deceleration biological information and the post-deceleration biological information to estimate whether or not the driver after deceleration is in a steady state.

**Amended claims under Art. 19.1 PCT**

1. A driver's state estimation apparatus comprising:

a biological information record unit configured to record biological information of a driver;

a deceleration start time detection unit configured to detect a deceleration start time when a vehicle in a travelling state starts to decelerate and stops;

a biological information extraction unit configured to refer to the biological information within a predetermined period recorded by the biological information record unit before the deceleration start time detected by the deceleration start time detection unit to extract pre-deceleration biological information which is a representative value of the referred biological information, and to refer to the biological information within a predetermined period recorded by the biological information record unit after the deceleration start time to extract post-deceleration biological information which is a representative value of the referred biological information; and

a driver's state estimation unit configured to compare the pre-deceleration biological information and the post-deceleration biological information extracted by the biological information extraction unit to estimate whether or not the driver after deceleration is in a steady state.

2. The driver's state estimation apparatus according to claim 1, wherein the driver's state estimation unit is configured to estimate that the driver after the deceleration is in the steady state when a difference between the pre-deceleration biological information and the post-deceleration biological information is larger than a predetermined difference threshold.

3. The driver's state estimation apparatus according to claim 1, wherein the biological information extraction unit is configured:

to set a time prior to the deceleration start time by a predetermined time as a pre-deceleration reference start time;
to set the deceleration start time as a pre-deceleration reference end time; and
to refer the biological information from the pre-deceleration reference start time to the pre-deceleration reference end time from the biological information recorded by the biological information record unit to extract the pre-deceleration biological information which is the presentative value of the referred biological information.

4. The driver's state estimation apparatus according to claim 1, wherein the biological information extraction unit is configured:

to set a time when a predetermined time elapses from the deceleration start time as a post-deceleration reference start time;
to set a time when a predetermined time elapses from the post-deceleration reference start time as a post-deceleration reference end time; and
to refer the biological information from the post-deceleration reference start time to the post-deceleration reference end time from the biological information recorded by the biological information record unit to extract the post-deceleration biological information which is the presentative value of the referred biological information.

5. The driver's state estimation apparatus according to claim 1, wherein the driver's state estimation unit is configured:

to set a time when a predetermined time elapses from the deceleration start time as a stability period reference start time;
to set a time when a predetermined time elapses from the stability period reference start time as a stability period reference end time;
to estimate that the driver after the deceleration is in the steady state when the difference between the pre-deceleration biological information and the post-deceleration biological information is larger than the predetermined difference threshold, and when an average change rate of the biological information from the stability period reference start time to the stability period reference end time referred from the biological information recorded by the biological information record unit is smaller than an average change ratio threshold.

6. The driver's state estimation apparatus according to claim 1, wherein the driver's state estimation unit is configured:

to set a time when a predetermined time elapses from the deceleration start time as a stability period reference start time;
to set a time when a predetermined time elapses from the stability period reference start time as a stability period reference end time;
to estimate that the driver after the deceleration is in the steady state when the difference between the pre-deceleration biological information and the post-deceleration biological information is larger than the predetermined difference threshold, and when a standard deviation of the biological information from the stability period reference start time to the stability period reference end time referred from the biological information recorded by the biological information record unit is smaller than the standard deviation threshold.

**7.** The driver's state estimation apparatus according to claim 1, wherein the biological information extraction unit is configured to refer the biological information within the predetermined period recorded before the deceleration start time by the biological information record unit to extract an average value of the referred biological information as the pre-deceleration biological information, and to refer the biological information within the predetermined period recorded after the deceleration start time by the biological information record unit to extract an average value of the referred biological information as the post-deceleration biological information.

**8.** The driver's state estimation apparatus according to claim 1, wherein the biological information record unit is configured to record a heart rate of the driver as the biological information.

**9.** The driver's state estimation apparatus according to claim 1, wherein further comprising a driver's information acquisition unit configured to acquire at least one of blood-pressure information, electrocardiogram information, heart rate information, respiratory information, pulse wave information, electrodermal activity information, body temperature information, facial expression information, and utterance information of the driver as driver's information, when it is estimated that the driver after the deceleration is in the steady state by the driver's state estimation unit.

**10.** The driver's state estimation apparatus according to claim 9, wherein further comprising a driver's information record unit configured to record the driver's information acquired by the driver's information acquisition unit in association with date-time information and current location information of the vehicle.

**11.** The driver's state estimation apparatus according to claim 1, wherein the deceleration start time detection unit is configured to preset a reaction time from a time when the driver recognizes that the vehicle needs to stop until a time when the driver starts a deceleration operation, and to detect a time prior to the time when the driver starts the deceleration operation by the reaction time is detected as the deceleration start time.

**12.** (Canceled)

**Statement under Art. 19.1 PCT**

Claim 12 is canceled.

# FIG. 1

```
11 ⌇  PULSE WAVE SENSOR           ────→  ┌──────────┐
12 ⌇  ELECTROCARDIOGRAPHIC        ────→  │          │  ⌇ 17
      MONITOR                             │          │
13 ⌇  VEHICLE SPEED SENSOR        ────→  │ CONTROLLER│
14 ⌇  BRAKE SWITCH                ────→  │          │
15 ⌇  ACCELERATION SENSOR         ────→  │          │
16 ⌇  NAVIGATION SYSTEM           ────→  └──────────┘
```

# FIG. 2

```
                              ⌇ 17
                22              23              24
                 ⌇               ⌇               ⌇
VEHICLE SPEED  ┌──────────┐  ┌──────────┐  ┌──────────┐
BRAKE        → │DECELERATION│→│BIOLOGICAL│→│DRIVER'S STATE│
ACCELERATION   │START TIME │  │INFORMATION│ │ESTIMATION UNIT│
               │DETECTION  │  │EXTRACTION │ └──────────┘
               │UNIT       │  │UNIT       │
               └──────────┘  └──────────┘
                                  ↑
                             ┌──────────┐
PULSE WAVE                   │BIOLOGICAL │
ELECTROCARDIOGRAPHIC      →  │INFORMATION│ ⌇ 21
WAVEFORM                     │RECORD UNIT│
                             └──────────┘
```

# FIG. 3

RISE IN BLOOD PRESSURE
DUE TO DECELERATION BEHAVIOR

BLOOD-PRESSURE

PRESENCE OR ABSENCE
OF DECREASE OF HEART RATE

HEART RATE

BRAKE

DECELERATION

VEHICLE SPEED

STOPPED

tb1    tg    ta1    ta2    TIME
       (tb2)

Tb    Tf    Ta
(Tr)        (Te)

EP 2 878 263 A1

| PRE-DECELERATION COMPARISON INTERVAL | POST-DECELERATION COMPARISON INTERVAL | STEADY STATE AFTER DECELERATION |

## FIG. 4A

BLOOD-PRESSURE

HEART RATE

Rb

Ra

$Rb-Ra \geqq Rt$

STEADY STATE

## FIG. 4B

BLOOD-PRESSURE

HEART RATE

Rb

Ra

## FIG. 4C

BLOOD-PRESSURE

HEART RATE

Rb

Ra

$Rb-Ra < Rt$

NOT STEADY STATE

## FIG. 4D

BLOOD-PRESSURE

HEART RATE

Rb

Ra

# FIG. 5

STRESS STATE ESTIMATION PROCESSING START

RECORD BIOLOGICAL INFORMATION — S101

V=0 — S102
- No
- Yes

tg IS UNREFERRED — S109
- No
- Yes

READ OUT tg — S110

SET Tb — S111

EXTRACT Rb — S112

SET Ta — S113

RESET Tb, Rb — S103

RESET Ta, Ra — S104

BRAKE ON — S105
- No
- Yes

ACCELERATOR ON — S100
- No
- Yes

tg IS UNRECORDED — S107
- No
- Yes

RESET tg — S106

RECORD tg — S108

Tf + Te ELAPSED — S114
- No
- Yes

EXTRACT Ra — S115

ΔR≧Rt — S116
- No
- Yes

STEADY STATE — S117

NOT STEADY STATE — S118

RESET Tb, Rb — S119

RESET Ta, Ra — S120

RETURN

EP 2 878 263 A1

# FIG. 6

RISE IN BLOOD PRESSURE
DUE TO DECELERATION BEHAVIOR

BLOOD-PRESSURE

HEART RATE

PRESENCE OR ABSENCE
OF DECREASE OF HEART RATE

BRAKE

VEHICLE SPEED

DECELERATION

STOPPED

tb1

tg'
(tb2)

ta1

ta2

TIME

Tb
(Tr)

Tf

Ta
(Te)

Tx

EP 2 878 263 A1

# FIG. 7

17

VEHICLE SPEED
BRAKE
ACCELERATION

| 22 | 23 | 24 |
| DECELERATION START TIME DETECTION UNIT | BIOLOGICAL INFORMATION EXTRACTION UNIT | DRIVER'S STATE ESTIMATION UNIT |

PULSE WAVE
ELECTROCARDIOGRAPHIC
WAVEFORM

21
BIOLOGICAL INFORMATION RECORD UNIT

25
DRIVER'S INFORMATION ACQUISITION UNIT

# FIG. 8A

Tp

PULSE WAVE

ELECTROCARDIOGRAPHIC WAVEFORM

t1   t2   TIME

# FIG. 8B

P

$P = \alpha \times Tp + \beta$

Tp

# FIG. 9

EP 2 878 263 A1

# FIG. 10

STRESS STATE ESTIMATION PROCESSING START

S101 — RECORD BIOLOGICAL INFORMATION

S102 — V=0 — Yes

No

S103 — RESET Tb, Rb

S104 — RESET Ta, Ra

No — BRAKE ON — S105

S100 — ACCELERATOR ON

No

Yes

S106 — RESET tg

Yes — S107 — tg IS UNRECORDED — No

S108 — RECORD tg

Yes

tg IS UNREFERRED — S109 — No

Yes

READ OUT tg — S110

SET Tb — S111

EXTRACT Rb — S112

SET Ta — S113

Th + Ti ELAPSED — S201 — No

Yes

EXTRACT Ra — S115

CALCULATE ρ AND σ — S202

ΔR≦Rt — S116 — No

Yes

ρ < ρt — S203 — No — S204

Yes

σ < σt — No

Yes

S117 — STEADY STATE

S118 — NOT STEADY STATE

ACQUIRE DRIVER'S INFORMATION — S205

RESET Tb, Rb — S119

RESET Ta, Ra — S120

RETURN

EP 2 878 263 A1

# FIG. 11

# FIG. 12

STRESS STATE ESTIMATION PROCESSING START

S101 — RECORD BIOLOGICAL INFORMATION

S102 — V=0 — Yes
No

S103 — RESET Tb, Rb

S104 — RESET Ta, Ra

S105 — BRAKE ON
No / Yes

S100 — ACCELERATOR ON
No / Yes

S107 — tg IS UNRECORDED
No / Yes

S106 — RESET tg

S108 — RECORD tg

S109 — tg IS UNREFERRED — No
Yes

S110 — READ OUT tg

S111 — SET Tb

S112 — EXTRACT Rb

S113 — SET Ta

S201 — Th + Ti ELAPSED — No
Yes

S115 — EXTRACT Ra

S202 — CALCULATE $\rho$ AND $\sigma$

S116 — $\Delta R \leqq Rt$ — No
Yes

S203 — $\rho < \rho t$ — No
Yes

S204 — $\sigma < \sigma t$ — No
Yes

S117 — STEADY STATE

S118 — NOT STEADY STATE

S205 — ACQUIRE DRIVER'S INFORMATION

S301 — RECORD DRIVER'S INFORMATION

S119 — RESET Tb, Rb

S120 — RESET Ta, Ra

RETURN

EP 2 878 263 A1

28

**EP 2 878 263 A1**

## INTERNATIONAL SEARCH REPORT

| | International application No. |
|---|---|
| | PCT/JP2013/003747 |

### A. CLASSIFICATION OF SUBJECT MATTER
*A61B5/18*(2006.01)i, *A61B5/0245*(2006.01)i, *B60R16/02*(2006.01)i

According to International Patent Classification (IPC) or to both national classification and IPC

### B. FIELDS SEARCHED

Minimum documentation searched (classification system followed by classification symbols)
A61B5/18, A61B5/0245, B60R16/02

Documentation searched other than minimum documentation to the extent that such documents are included in the fields searched

| | | | |
|---|---|---|---|
| Jitsuyo Shinan Koho | 1922–1996 | Jitsuyo Shinan Toroku Koho | 1996–2013 |
| Kokai Jitsuyo Shinan Koho | 1971–2013 | Toroku Jitsuyo Shinan Koho | 1994–2013 |

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| A | JP 2003-61921 A (Mitsuba Corp.), 04 March 2003 (04.03.2003), entire text; all drawings (Family: none) | 1-11 |
| A | Motohiro TAKIZAWA et al., "The Psychological Influence on the Driver by the Change of Running Circumstance", The Japan Society of Mechanical Engineers Kotsu·Butsuryu Bumon Taikai Koen Ronbunshu, vol.12th, 08 December 2003 (08.12.2003), 233 to 236 | 1-11 |
| A | JP 2009-213768 A (Denso Corp.), 24 September 2009 (24.09.2009), entire text; all drawings (Family: none) | 1-11 |

☐ Further documents are listed in the continuation of Box C.   ☐ See patent family annex.

| | |
|---|---|
| * Special categories of cited documents: <br> "A" document defining the general state of the art which is not considered to be of particular relevance <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |

| Date of the actual completion of the international search <br> 25 June, 2013 (25.06.13) | Date of mailing of the international search report <br> 09 July, 2013 (09.07.13) |
|---|---|
| Name and mailing address of the ISA/ <br> Japanese Patent Office | Authorized officer |
| Facsimile No. | Telephone No. |

Form PCT/ISA/210 (second sheet) (July 2009)

## INTERNATIONAL SEARCH REPORT

| International application No. |
| --- |
| PCT/JP2013/003747 |

---

**Box No. II    Observations where certain claims were found unsearchable (Continuation of item 2 of first sheet)**

This international search report has not been established in respect of certain claims under Article 17(2)(a) for the following reasons:

1. ☒ Claims Nos.: 12
     because they relate to subject matter not required to be searched by this Authority, namely:

     In the invention described in claim 12, it is presumed whether or not a driver is in a resting state. Thus, the invention described in this claim includes a step of determining a physical state, and therefore pertains to a method for diagnosis of the human body.

2. ☐ Claims Nos.:
     because they relate to parts of the international application that do not comply with the prescribed requirements to such an extent that no meaningful international search can be carried out, specifically:

3. ☐ Claims Nos.:
     because they are dependent claims and are not drafted in accordance with the second and third sentences of Rule 6.4(a).

---

**Box No. III    Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.    Consequently, this international search report is restricted to the invention first mentioned in the claims; it is covered by claims Nos.:

**Remark on Protest**    ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007047196 A

- JP 2012166225 A